# EUROPEAN PATENT SPECIFICATION

(11) **EP 0 921 778 B1**
(45) Date of publication and mention of the grant of the patent: **12.03.2003**
(21) Application number: 97934189.8
(22) Date of filing: 09.07.1997
(51) Int. Cl.: A61F 13/20, A61F 13/22

(54) **TAMPON HAVING A RESILIENT MEMBER AND A METHOD OF FORMING THE TAMPON**
TAMPON MIT ELASTISCHEM TEIL UND VERFAHREN ZU SEINER HERSTELLUNG
TAMPON POSSEDANT UN ELEMENT ELASTIQUE ET SON PROCEDE D'ELABORATION

(30) Priority: 12.08.1996 US 695488; 12.08.1996 US 693686
(43) Date of publication of application: 16.06.1999
(73) Proprietor: KIMBERLY-CLARK WORLDWIDE, INC., Neenah, Wisconsin 54956 (US)
(72) Inventor: ACHTER, Amy, Michele, Neenah, WI 54956 (US); BALZAR, Tammy, Jo, Menasha, WI 54952 (US); FOX, Donald, George, Neenah, WI 54956 (US)
(74) Representative: Diehl, Hermann, Dr. Dipl.-Phys.
(86) International application number: US9712447
(87) International publication number: WO98006365

(56) References cited:
- US-A- 3 469 286
- US-A- 3 643 661
- US-A- 3 683 915
- US-A- 3 706 311
- US-A- 3 731 687
- US-A- 3 971 378
- US-A- 4 212 301

## Description

### FIELD OF THE INVENTION

This invention relates to a tampon having a resilient member. The invention also relates to a method of forming the tampon. More specifically, this invention relates to a catamenial tampon having a resilient member which is designed to prevent premature leakage of body fluid from a woman's vagina when the tampon is initially inserted into the woman's vagina. The invention also promotes more efficient material utilization during the use of the tampon.

### BACKGROUND OF THE INVENTION

Currently, there are two basic types of catamenial tampons used for feminine hygiene. The first type is a digital tampon which is designed to be inserted into a woman's vagina directly by the user's fingers. The second type is a tampon which is designed to be inserted with the aid of an applicator. Both types are usually made by folding or rolling a loosely associated strip of absorbent material into an elongated shape often referred to as a "softwind." The softwind is then radially and/or biaxially compressed into a pledget. The pledget may or may not include a cover. In both types of tampons, a withdrawal string is attached to the softwind, either before or after compression, to facilitate removal of the tampon from the user's vagina after it has absorbed a certain quantity of body fluid, such as menses, blood, etc.

It has been found that many tampons, both digital as well as those delivered by an applicator, are unable to prevent premature leakage of body fluid. Premature fluid leakage can result from a number of factors. One factor is that the tampon does not properly fit above the introital region of the vagina. A second factor is that the tampon has been compressed to such an extent that it is unable to open or radially expand rapidly enough after initial insertion into a woman's vagina to absorb the body fluid which comes into contact with it. A third factor is that the tampon is not shaped correctly to intercept fluid flow through the vaginal canal. A fourth factor is that the folds and convolutions of the vagina are not in contact with the tampon and therefore body fluid is able to bypass the tampon. US 3469286 discloses a tampon and a method for making compressed cata menial tampons in which a thick mat of absorbent material is compressed to form a thin sheet which is cut into narrow strips which is folded in one of several spring-like configurations. A withdrawal string is attached to the posterior end or to the anterior portion or to a central portion. US 3706311 discloses a self-spreading catamenial tampon made from absorbent material.

Now a catamenial tampon has been invented which has a resilient feature which will prevent premature leakage of body fluid immediately after being inserted into a woman's vagina. A method of forming the tampon is also described.

### SUMMARY OF THE INVENTION

Briefly, this invention relates to a catamenial tampon. The tampon includes an absorbent and a resilient member which is positioned on the absorbent. The absorbent and resilient member are formed into an elongated softwind having a first end and a second end. The softwind is folded upon itself such that the first and second ends are aligned adjacent one another and the softwind contains at least two folds therebetween. The softwind is then compressed into an elongated pledget having an insertion end and a trailing end with the insertion end containing more absorbent than the trailing end. The resilient member is capable of expanding at least a portion of the pledget so as to intercept fluid flow when the tampon is inserted into a woman's vagina. The tampon further includes withdrawal means for removing the tampon from a woman's vagina. The withdrawal means is attached to the first and second ends of the pledget.

The method includes the steps of assembling a resilient member and an absorbent to form a structure having a central longitudinal axis X--X. The resilient member can either be aligned with or offset from the central longitudinal axis X--X. The absorbent and the resilient member are then rolled or folded up into an elongated softwind which has a first end and a second end. The elongated softwind is then folded such that the first and second ends are aligned adjacent one another and the softwind contains at least two folds therebetween. The softwind is then compressed into a pledget having an insertion end and a trailing end. A withdrawal string is then secured to the first and second ends of the pled get to form a tampon. The withdrawal string facilitates removal of the tampon from a woman's vagina.

The general object of this invention is to provide a tampon for absorption of menses, blood, etc. A more specific object of this invention is to provide a catamenial tampon having a resilient feature which is designed to prevent premature leakage of body fluid when initially inserted into a woman's vagina.

Another object of this invention is to provide a method of forming a tampon having a resilient member.

Another object of this invention is to provide a tampon which springs open immediately upon insertion into a woman's vagina to prevent bypass of menses and other body fluids.

A further object of this invention is to provide a tampon which contains a resilient member which has the potential to spread open at least a portion of the tampon so that the tampon will contact a larger cross-section of a woman's vagina thereby preventing the bypass of menses and other body fluid.

Still another object of this invention is to provide a tampon which has dry expansion capability.

Still further, an object of this invention is to provide a tampon which exhibits reduced slippage during use.

Still further, an object of this invention is to provide a tampon which is expandable to fit within the non-circular cross-section of a woman's vagina.

Still further, an object of this invention is to provide a tampon which better utilizes the absorbent during use.

Other objects and advantages of the present invention will become more apparent to those skilled in the art in view of the following description and the accompanying drawings.

### BRIEF DESCRIPTION OF THE DRAWINGS

Fig. 1 is a perspective view of a resilient member positioned on an absorbent and aligned along the central longitudinal axis X--X thereof.
Fig. 2 is a perspective view of the absorbent and resilient member shown in Fig. 1 after being rolled or folded into a softwind.
Fig. 3 is a perspective view of an alternative embodiment depicting an absorbent, cover and resilient member being aligned such that the resilient member is offset from the central longitudinal axis X--X.
Fig. 4 is a perspective view of the absorbent, cover and resilient member shown in Fig. 3 rolled up into a softwind.
Fig. 5 is a side view of the softwind folded into a generally M-shaped profile with the first and second ends being aligned adjacent to one another and retained by the withdrawal string.
Fig. 6 is a side view of the softwind shown in Fig. 5 after it has been compressed into a pledget.
Fig. 7 is a side view of the tampon showing the configuration it attempts to acquire after it has been inserted into a woman's vagina.
Fig. 8 is a side view of the tampon showing one possible configuration during removal from a woman's vagina.
Fig. 9 is a side view of a two piece tampon applicator having an inner tube and an outer tube.
Fig. 10 is a cross-sectional view of the tampon applicator shown in Fig. 9 depicting the tampon being retained in the outer tube prior to insertion into a woman's vagina.
Fig. 11 is a flow diagram of a method of forming a tampon.
Fig. 12 is a flow diagram of an altemative method of forming a tampon.

### DETAILED DESCRIPTION OF THE PREFERRED EMBODIMENTS

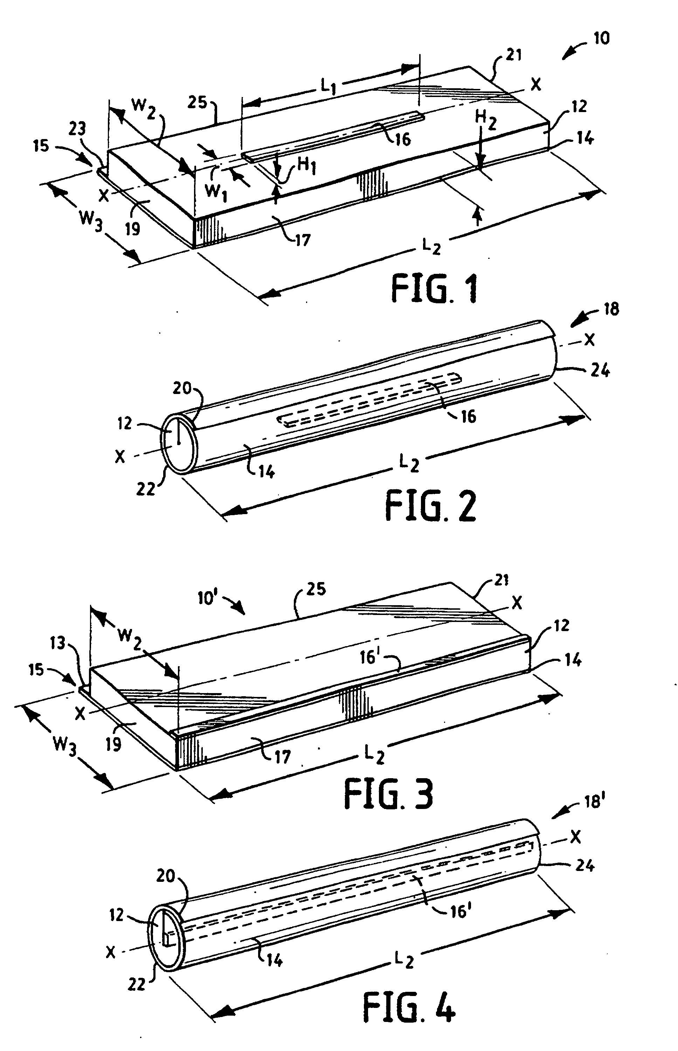

Referring to Fig. 1, one possible way of forming a catamenial tampon 10 is shown which is useful for absorbing body fluid from a woman's vagina, especially during her menstrual cycle. The tampon 10 is designed to be inserted above the introital region of a woman's vagina and is designed to function so as to intercept the fluid flow of menses blood, and other body fluids and prevent the fluid from exiting the vagina. The tampon 10 includes an absorbent 12 which may contain a cover 14. The absorbent 12 can be formed from absorbent fibers which are assembled into an absorbent sheet or ribbon. Altematively the absorbent 12 can be formed from absorbent fibers which are assembled and compressed into a generally elongated and/or cylindrical configuration. The absorbent 12 is preferably formed from cellulosic fibers, such as cotton and rayon. The absorbent can be 100% cotton, 100% rayon, or a blend of cotton and rayon fibers. Some blends which have been tried and work well include a blend of about 15% cotton to about 85% rayon; about 70% cotton to about 30% rayon; about 60% cotton to about 40% rayon; about 25% cotton to about 75% rayon; and about 6% cotton to about 94% rayon. The particular blend of fibers can vary depending upon one's preference.

When cotton fibers are used, the cotton fibers should have a staple length of between about 5 millimeters (mm) to about 20 mm. The cotton fibers should generally have a fiber size of between about 150 µm (microns) to about 280 µm (microns). The cotton fibers can also be bleached if desired. Bleaching will make the cotton fibers whiter in appearance.

When rayon fibers are present, the rayon fibers should have a staple length of between about 20 mm to about 35 mm. The rayon fibers should have a denier of between about 2 to about 6. Denier is a unit of fineness of yam based on a standard of 50 milligrams (mg) for 450 meters of yam. The rayon fibers can have a circular, a bi-lobal, a tri-lobal cross-sectional configuration, or some other cross-sectional configuration known to those skilled in the art. The bi-lobal configuration has a cross-sectional profile which looks like a dog bone while the tri-lobal configuration has a cross-sectional profile which looks like a "Y". The rayon fibers can also be bleached if desired.

The absorbent 12, when fbrmed from an absorbent sheet or ribbon, is constructed from a blend of cotton and rayon fibers. Two processes for forming such an absorbent sheet are known as "carding" and "airlaying." Depending upon the desired absorbency one desires in the finished tampon, the basis weight of the absorbent sheet can vary. The U.S. Food and Drug Administration (FDA) has set absorbency standards for "junior", "regular", "super" and "super-plus" size tampons. In order to meet the FDA standards for these four sizes, the absorbent sheets are targeted to have basis weights of about 100 grams per square meter (gsm), 120 gsm, 170 gsm and 230 gsm, respectively. Typically, the carding process is controlled to produce an absorbent sheet with a width of between about 40 mm to about 60 mm, preferably about 50 mm. The basis weight and/or the length of the absorbent 12 can also be adjusted to form the different size tampons.

The absorbent 12 can be partially or fully enclosed by a cover 14, preferably a liquid permeable cover. By "liquid-permeable" it is meant that body fluid is able to pass through the cover 14 in a quick and efficient manner. The cover 14 can be hydrophilic or hydrophobic. By "hydrophilic" it is meant that the cover 14 has an affinity for absorbing or tending to combine with water. By "hydrophobic" it is meant the cover 14 is antagonistic to or tending not to combine with water. The cover 14 can also be treated with a surfactant or other material to make it hydrophilic or to make it more hydrophilic.

The liquid-permeable cover 14 can be formed from woven or nonwoven materials having a porous substrate. Woven materials include textile fabrics which can be made from rayon, cotton or polyolefins. The polyolefins can be either staple or continuous filaments. The nonwoven materials can include spunbond, bonded carded webs and hydroentangled webs. Spunbond and bonded carded webs are commercially sold by Kimberly-Clark Corporation having an office at 401 N. Lake Street, Neenah, WI 54956. Another nonwoven material which can be used as the cover 14 is formed from 100% polyester fibers held together by a binder. This material is known as powder-bonded-carded web (PBCW). PBCW is commercially available from HDK Industries, Inc. having an office at 304 Arcadia Drive, Greenville, South Carolina 29609. The cover 14 can further be formed from an apertured thermoplastic film having either a two-dimensional or a three-dimensional thickness. Apertured thermoplastic films are available from several commercial vendors. Two such vendors include Pantex Srl, Pantex Sud srl, Via Terracini snc, having an office at 51031 Agliana, Pistoia, Italy and Applied Extrusion Technology having a mailing address of P.O. Box 582, Middleton, Delaware 19709.

The liquid-permeable cover 14 can be treated with an aqueous solution to reduce frictional drag, to give the tampon 10 a permanent wettability and to enhance the ease of insertion into and withdrawal from a woman's vagina. The cover 14 can be treated either before being rolled or folded up with the absorbent 12 and a resilient member 16 or after a softwind 18 has been formed. The different types of aqueous solutions which can be used are known to those skilled in the art. One particular type of aqueous solution is taught in U.S.S.N. 08/311.692. filed September 23, 1994 and entitled: "TAMPON EXHIBITING LOW FRICTIONAL DRAG."

The tampon 10 further includes a resilient member 16 which is positioned on the top surface of the absorbent 12. The three vertically stacked members, from top to bottom, include the resilient member 16, the absorbent 12 and the liquid-permeable cover 14. The three members are shown having a rectangular configuration and being symmetrically aligned relative to a central longitudinal axis X--X. It should be noted that the absorbent 12 and cover 14 could have different configurations if desired, for example, an hourglass configuration. The resilient member 16 should be capable of having what is known as "dry and wet expansion" characteristics. In other words, the resilient member 16 should be made of a material which is capable of expanding back to or towards its original configuration in either a dry state, a wet state, and preferably, in both a dry and a wet state. Dry expansion of the resilient member 16 is beneficial in that the tampon 10 does not have to be wetted by body fluid before the resilient member 16 is capable of expanding. Furthermore, it is advantageous to employ a material for the resilient member 16 which is capable of expanding even while the tampon 10 is wetted by body fluid. For purposes of this invention, the resilient member 16 can be a resilient foam, such as a closed cell foam or an open cell foam. Other materials from which the resilient member 16 can be made from include polyethylene oxide (PEO) and polyvinyl alcohol (PVA). The resilient member 16 can also be formed from resilient fibers such as polyolefin based fibers, polyethylene oxide fibers, hydrophobic rayon fibers and the like which preferably will have characteristics similar to those of a resilient foam. The resilient member 16 can also be made or constructed from a wettable foam. An open cell foam which works well and has good resilient properties is commercially available under the trade name ACQUELL and is sold by Sentinel Products Corporation having an office located at 70 Airport Road, Hyannis, Massachusetts 02601. A polyethylene dosed cell foam having good flexibility characteristics also works well. This foam is commercially sold under the tradename "VOLARA" and is available from Voltex, a Division of Sekisui America Corporation, having an office located at 100 Shepard Street, Lawrence, Massachusetts 01843.

The resilient member 16 can have a square, rectangular, oval, circular, or any other desired cross-sectional configuration. Preferably, the resilient member 16 will have a uniform thickness and width. If desired, the dimensions of the resilient member 16 do not have to be uniform. As depicted in Fig. 1, the resilient member 16 has a length L₁ which is less than the length L₂ of the absorbent 12. The width W₁ of the resilient member 16 is less than the width W₂ of the absorbent 12. It has been found that a resilient member 16 having a length of between about 1 inch to about 5 inches (about 25.4 mm to about 127 mm), a width W₁ of between about .25 inches to about .50 inches (about 6.3 mm to about 12.7 mm), and a height H₁ of between about 1/16 of an inch to about 1/8 of an inch (about 1.6 mm to about 3.2 mm) works well when positioned on an absorbent 12 having a length L₂ of from between about 3 inches to about 10 inches (about 76 mm to about 254 mm), a width W₂ of from between about 1 inch to about 3 inches (about 25.4 mm to about 76 mm), and a height H₂ of from between about 1/8 of an inch to about 1 inch (about 3.2 mm to about 25.4 mm).

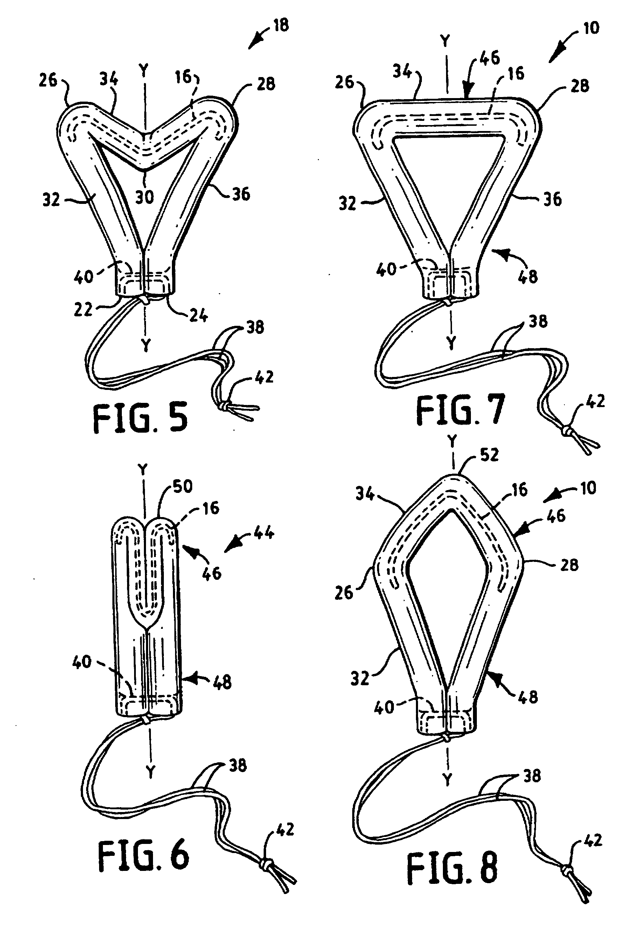

The resilient member 16 can have a length L₁ which is only a fraction of the length L₂ of the absorbent 12. Alternatively, the resilient member 16 can have a length L₁ which is equal to the length L₂ of the absorbent 12. In Fig. 1, the resilient member 16 is shown having a length L₁ which is equal to about 1/2 of the length L₂ of the absorbent 12. It should be noted that the resilient member 16 should have a length L₁ which is at least 1/3 of the length L₂ of the absorbent ribbon 12, and preferably, at least 1/2 of the length L₂ of the absorbent 12. Alternatively, the resilient member 16 or 16' can have a length L₁ which is equal to or approximately equal to the length L₂ of the absorbent 12, as is depicted in Fig. 3.

Referring to Fig. 2, the absorbent 12, the cover 14 and the resilient member 16 are shown after they have been rolled or folded into a generally elongated member often referred to as a "softwind" 18. The softwind 18 has a central longitudinal axis X--X and is an elongated, tubular shaped member. The softwind 18 can have a circular, square, rectangular or other cross-sectional profile but preferably is elongated and has a cylindrical shape. The softwind 18 can have a length of up to about 10 inches (about 254 mm). Preferably, the softwind 18 has a length of from between about 2 inches to about 8 inches (about 51 mm to about 203 mm), and most preferably, it has a length of about 6 inches (about 152 mm). The length of the softwind can be varied so as to obtain the various size tampons.

When the softwind 18 has a circular cross-section, it can have a diameter ranging from between about 0.5 inches to about 2 inches (about 12.7 mm to about 51 mm), and preferably, will have a diameter of about 1 inch (about 25.4 mm). The specific diameter of the softwind 18 will be dictated by the type of tampon which is to be formed, such as a "junior", "regular", "super", or "super-plus" size tampon. The softwind 18 is not compressed.

When the absorbent 12, the cover 14 and the resilient member 16 are rolled into the softwind 18, the cover 14 can have a width W₃ which is equal to or greater than the width W₂ of the absorbent 12. A wider width for the cover 14 is preferred for it enables the cover 14 to be wrapped around the exterior surface of the absorbent 12 and overlap upon itself, as indicated at 20. If the width W₃ of the cover 14 is equal to the width W₂ of the absorbent 12 then the ends of the cover 14 will abut one another. The overlap design for the cover 14 is preferred.

Referring again to Fig. 2, the absorbent 12, the cover 14 and the resilient member 16 are rolled up or folded into the softwind 18 which has a first end 22 and a second end 24. The first and second ends, 22 and 24 respectively, are spaced apart from each other. Since the resilient member 16 is spaced inward from the first and second ends, 22 and 24 respectively, the resilient member 16 is not visible when the softwind 18 is formed. In forming the softwind 18, the cover 14 is depicted as being overlapped upon itself at 20 and can be bonded to itself and/or to the absorbent 12. It should be again mentioned that if the softwind 18 can be formed from the absorbent 12 alone, no cover 14 need be present. The cover 14 can be bonded using heat, pressure, heat and pressure, ultrasonics, an adhesive such as glue, or any other known bonding technique. Alternatively, the cover 14 could be sprayed onto the absorbent 12. The softwind 18 will have an length L₂ which will be equal to the length L₂ of the absorbent 12 and the cover 14. Should the cover 14 have a longer length than the absorbent 12, then the softwind 18 would have a length equal to the cover 14, unless the ends of the cover 14 are tucked at each end. The length of the cover 14 could also be less than L₂ to allow for cover drift during manufacture of the softwind 18.

Referring to Figs. 3 and 4, an alternative embodiment is shown wherein a tampon 10' is formed by positioning a rectangular sheet of an absorbent 12 over a rectangular sheet of a liquid-permeable cover 14 and then positioning a resilient member 16' on the upper surface of the absorbent 12. The absorbent 12 and the cover 14 both have an equal length L₂ which is equal to the length of the resilient member 16'. The absorbent 12 has a width W₂ which is slightly smaller than the width W₃ of the cover 14. The resilient member 16' is offset from the central longitudinal axis X--X such that it is located adjacent to an outside edge of the absorbent 12. In this configuration, it is easier to roll up the absorbent 12, the cover 14 and the resilient member 16' to form an elongated generally cylindrically shaped softwind 18'. This embodiment also shows that the resilient member 16' extends from the first end 22 to the second end 24 of the softwind 18'. In this design, the resilient member 16' is visible at each end 22 and 24 of the softwind 18'. This configuration may be advantageous in that the resilient member 16' may be able to outwardly expand the tampon 10' more fully when the tampon 10' is initially inserted into the woman's vagina. The extra length of the resilient member 16' may allow for a more positive expansion. This embodiment may also be better at preventing or reducing slippage of the tampon 10' after it has been inserted above the introital region of a woman's vagina, thereby providing superior leakage protection. Another attribute of this embodiment is that the resilient member 16' may only have to have a portion of the resilient characteristic exhibited by the shorter resilient member 16. Comfort to the consumer can thereby be enhanced by using a resilient member 16' which does not have as much strength in causing the tampon 10' to expand outward and span across the walls of a woman's vagina.

Referring to Fig. 5, the softwind 18 is folded or bent upon itself such that the first and second ends, 22 and 24 respectively, are aligned adjacent to one another and the softwind 18 contains at least two folds 26 and 28 located therebetween. By being aligned "adjacent to one another" it is meant that the first and second ends, 22 and 24 respectively, are positioned side by side, parallel to one another, offset axially or spaced radially apart from one another, or are positioned in some other type of arrangement whereby the first and second ends, 22 and 24 respectively, are close to one another. Preferably, the softwind 18 will contain three folds 26, 28 and 30 which are located between the first and second ends, 22 and 24 respectively, to give the softwind 18 a generally M-shaped profile. In the generally M-shaped profile, the softwind 18 is folded at 26 to form a first portion 32 and is folded a second time at 28 to form a second portion 34 and a third portion 36. The first and third portions, 32 and 36 respectively, preferably have approximately the same length, although their lengths can differ as desired. It is also possible to form the first, second and third portions 32, 34 and 36 to have approximately the same length. For example, if the softwind 18 has a length of about 6 inches (about 152 mm), each of the portions 32, 34 and 36 can have a length of approximately 2 inches (about 51 mm). The resilient member 16 should have a length such that it can extend between the first and second folds, 26 and 28 respectively. Preferably, the resilient member 16 will extend along the length of the second portion 34 and will have a sufficient length such that it will extend beyond the first and second folds, 26 and 28 respectively. It has been found that when the resilient member 16 extends beyond the folds 26 and 28 by at least an 1/8 of an inch (about 3.2 mm), and preferably, by at least a 1/4 of an inch (about 6.4 mm), that the resilient member 16 becomes more effective in causing the second portion 34 to span laterally outward once the tampon 10 is inserted into a woman's vagina.

Referring again to Fig 5, the third fold 30 provides the softwind 18 with the generally M-shaped profile. The third fold 30 can be positioned an equal or an unequal distance between the first and second folds, 26 and 28 respectively. When the third fold 30 is positioned an equal distance between the first and second folds, 26 and 28 respectively, the third fold 30 will be axially aligned along a central longitudinal axis Y--Y, as is shown in Fig. 5. The central longitudinal axis Y--Y vertically divides the generally M-shaped profile of the softwind 18 into left and right mirror images. It is also possible to form the third fold 30 closer to either the first or second folds, 26 and 28 respectively, if desired. The third fold 30 causes the second portion 34 of the resilient member 16 to be folded into two parts such that a V-shape is obtained for the second portion 34.

The tampon 10 further includes a withdrawal string 38 for assisting in removing the tampon 10 from the woman's vagina. The withdrawal string 38 is attached to the absorbent 12, and preferably, to the first and second ends, 22 and 24 respectively, of the softwind 18. One method of attaching the withdrawal string 38 is to form an aperture or hole 40 through the first and third portions 32 and 36, approximate the first and second ends, 22 and 24 respectively. The withdrawal string 38 is then threaded through the aperture 40 and looped upon itself so as to cinch it secure to the absorbent 12. The free ends of the withdrawal string 38 are then tied in a knot 42 to assure that the withdrawal string 38 will not separate from the softwind 18. The knot 42 also serves to prevent fraying of the withdrawal string 38 and to provide a place or point where a woman can grasp the withdrawal string 38 when she is ready to remove the tampon 10 from her vagina. It should be noted that the withdrawal string 38 holds the first and second ends, 22 and 24 respectively, in direct contact with one another and will limit the amount they can expand while positioned within the woman's vagina. It should be noted that the withdrawal string 38 can be secured to and/or attached to various areas of the softwind 18 and can pass through either the absorbent 12, the cover 14 or both. The withdrawal string 38 can also be attached either before the softwind 18 is compressed or after the softwind 18 is compressed.

The withdrawal string 38 can be constructed from various types of threads or ribbons. A thread or ribbon made from 100 percent cotton fibers works well. The withdrawal string 38 should have a length which extends beyond the end of the tampon 10 from between about 2 inches to about 8 inches (about 51 mm to about 203 mm), preferably from about 4 inches to about 6 inches (about 102 mm to 152 mm), and most preferably, about 5 inches (about 127 mm). The withdrawal string 38 can be dyed and/or treated with an anti-wicking agent, such as wax, before being secured to the softwind 18. The anti-wicking agent will facilitate and prevent body fluids from wicking along the withdrawal string 38 and contacting the inner surface of a woman's undergarment. A dry, clean withdrawal string 38 is preferred by the user, especially when she goes to remove the tampon 10 from her vagina.

Referring to Fig. 6, the softwind 18 is compressed into a pledget 44 having an insertion end 46 and a trailing end 48. The pledget 44 can have any desired shape but preferably, it will have a generally cylindrically-shape with a circular cross-section configuration or a generally elongated shape with a rectangular cross-sectional configuration. The insertion end 46 is designed to be the first part of the pledget 44 which enters the woman's vaginal cavity. It should be noted that, while in use, the pledget 44 will be entirely positioned within the woman's vagina. The insertion end 46 contains a cleft 50 formed by the fold 30. Because the softwind 18 has a generally M-shaped profile, the insertion end 46 can contain an equal amount and, preferably, a greater amount of absorbent material than the trailing end 48. When a greater amount of absorbent material is present at the insertion end 46 and the outside diameter of the insertion end 46 is approximately equal to the outside diameter of the trailing end 48, the amount of absorbent material in the insertion end 46 will have to be densified to a greater extent than the absorbent material making up the trailing end 48. By having a greater amount of absorbent material present at the insertion end 46, the tampon 10 is better able to absorb body fluid and prevent premature leakage. When the pledget 44 is formed, the absorbent 12, the cover 14 and the resilient member 16 are all compressed. However, the compression step should not detrimentally effect the function of the resilient member 16. In other words, the resilient member 16 has to be capable of expanding outward towards or to its original configuration once the tampon 10 or 10' is inserted into the woman's vagina.

Referring to Fig. 7, the tampon 10 is shown in its expanded condition which occurs immediately upon its insertion into a woman's vagina. In Fig. 7, one can see that the resilient member 16 has expanded the second portion 34 such that the third fold 30 has disappeared and the first and third portions, 32 and 36 respectively, are forced outwardly towards the interior walls of the woman's vagina. The resilient member 16 is capable of expanding laterally outward towards its original position such that at least a portion of the tampon 10 is expanded outward to intercept fluid flow through the woman's vagina. In the expanded condition, the tampon 10 will take on a generally triangular shape which is closed at the bottom or trailing end 48 by the attachment of the withdrawal string 38.

While within the vaginal cavity, the resilient member 16 will expand thereby causing the first and third portions, 32 and 36 respectively, to spring or expand outward and spread across the vaginal opening. The tampon 10 will usually be positioned below the cervix and therefore should be in a position to capture any menses, blood, and/or mucus-like clots discharged by the woman's body. The resilient, elastic and flexible characteristics of the resilient member 16 enables it to recover quickly from its compressed and deformed shape. This allows the tampon 10 to conform more ideally to the vaginal opening and press against the inside walls and convolution of a woman's vagina. This action will prevent the premature leakage of body fluid due to improper fit or the inability of the absorbent material of the tampon 10 to expand outward rapidly enough to catch any body fluid which may initially contact the tampon 10. This tampon design will also provide better utilization of the absorbent 12 during use.

The resilient member 16 also serves to reduce and/or prevent slippage of the tampon 10 within the vaginal canal. The vaginal canal is normally in a position of collapse in its lower portion for 3/4 to 4/5 of its length. The lower 1/3 is essentially always dosed and its inside walls are aligned side by side or next to each other as a result of the action of the muscular tissue of the vagina. As soon as the tampon 10 is inserted beyond the lower portion of the vaginal canal, the muscular tissue causes the inside walls to close off the vaginal mouth so that the tampon 10 can rest above this lower portion of the vagina. It will therefore be self-evident that the tampon 10 cannot be dislodged through movement of the wearer's body. This is an important feature of this invention.

Referring again to Fig. 7, one can see that the resilient member 16 is strong enough to cause the absorbent 12 to straighten out in the area where the third fold 30 was present. Preferably, the second portion 34 will straighten out towards its original profile and the third fold 30 will disappear upon expansion of the tampon 10 within the woman's vagina. By expanding the second portion 34 so that it bridges across the vagina opening, one can see that the tampon 10 is in a much better position to absorb the influx of menses and/or blood that may contact it after it has been inserted into the woman's vagina. It should also be recognized that by extending the length of the resilient member 16 around the locations of the first and second folds, 26 and 28 respectively, that the tampon 10 can open within the vagina while retaining a smooth curvature approximate first and second folds, 26 and 28 respectively. This smooth curvature at the locations of the first and second folds, 26 and 28 respectively, assures that the woman will not experience any discomfort during use. The presence of the resilient member 16 is also beneficial in that it does away with any required conditioning period wherein the absorbent 12 requires a certain amount of time before it can radially expand outward and intercept an appreciable amount of body fluid. Many prior art tampons are incapable of preventing leakage of body fluid until the absorbent has expanded a certain amount so as to fill the vaginal passage.

Referring to Fig. 8, one can see that upon removal of the tampon 10 from the woman's vagina, that the second portion 34 may bow outward as the first and second folds, 26 and 28 respectively, are pressed inward by the interior walls of the woman's vagina. This action is caused by pulling downward on the withdrawal string 38. As the withdrawal string 38 is pulled downward, the first and third portions, 32 and 36 respectively, are forced inward towards one another. This action will allow the first and second folds, 26 and 28 respectively, to move closer together and will urge the second portion 34 to bow outward and form an apex 52. As this occurs, the outside diameter of the tampon 10 is significantly reduced in size and a comfortable removal of the tampon 10 is possible. It should be noted that sometimes the second portion 34 will fold inward to the configuration shown in Fig. 5 upon removal from the vagina. This configuration also allows for a comfortable removal for the first and third portions, 32 and 36 respectively, are able to move closer together as the withdrawal string 38 to pulled downward.

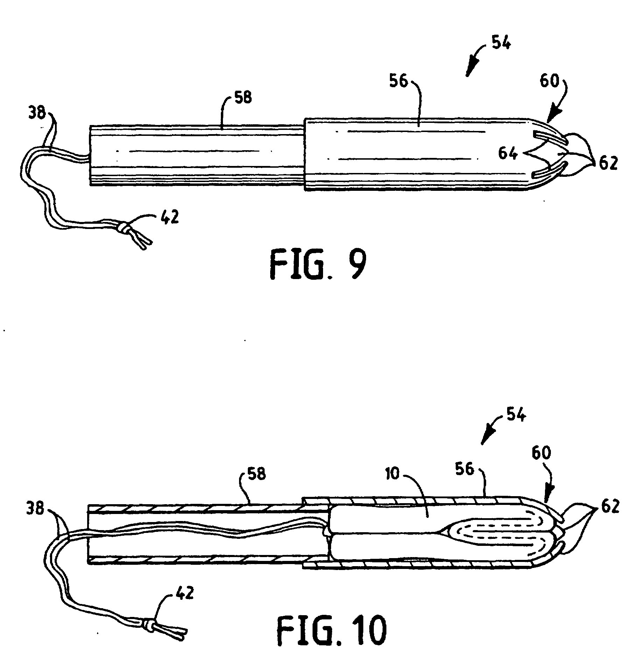

Referring now to Figs. 9 and 10, a tampon applicator 54 is shown which includes an outer tube 56 and an inner tube 58. Both of the tubes 56 and 58 are hollow and each one can be formed from paper, paper board, cardboard, plastic, thermoplastic film, a combination thereof or from other known materials. The outer tube 56 and the inner tube 58 can be formed of similar or dissimilar materials, but preferably both are formed from the same material. If paper, paper board or cardboard is used, it can be coated with a wax or water-insoluble polymer to render it water-resistant. Suitable plastic materials include polyolefins, such as low density polyethylene and polypropylene. The outer tube 56 should have sufficient strength and rigidity to prevent collapsing under normal vaginal pressures. The outer tube 56 and the inner tube 58 can be formed into a cylindrical shape having a longitudinal seam or be spirally or convolutely wound. The outer tube 56 can have a relatively small diameter of between from about 10 mm to about 20 mm if desired. The inner tube 58 should be slightly smaller in diameter than the outer tube 56 and is designed to telescopically slide within the inner diameter of the outer tube 56.

The applicator 54 also has an insertion tip 60 formed at one end of the outer tube 56 which can contain a plurality of soft and flexible petals 62. The petals 62 can be arranged to form a dome-shaped nose. The petals 62 can be separated by narrow slots 64. Perforation can be substituted for the slots 64 if desired. The slots 64 allow each petal 62 to radially flex or bend outward so as to provide an enlarged opening through which the tampon 10 can be expelled as the inner tube 58 is pushed into the outer tube 56. Either an even or an odd number of petals 62 can be used, but preferably, there are an odd number of petals 62, such as 3, 5, 7, etc. By using an odd number of petals 62, one can prevent the outer tube 56 from collapsing or flattening after the tampon 10 has been expelled. Most preferably, the tampon applicator 54 will contain five petals 62. By preventing the outer tube 56 from collapsing, one can be assured that the tissues of the woman's vagina will not be pinched when the tampon applicator 54 is inserted into or removed from the woman's vagina. For optimum performance, all of the petals 62 should have the same shape and dimension. Each of the petals 62 can have an elongated, approximately truncated shape, with a rounded end and each can have a length of about 7/16 of an inch (about 11.1 mm).

The tampon applicator 54 is very useful in that it will assure that the compressed tampon 10 will retain its unique shape until it is inserted into a woman's vagina. At that time, without the restriction of the outer tube 56, the resilient member 16 will be able to expand outward towards its original position such that the first and third portions, 32 and 36 respectively, will move outward towards the inner walls of the vagina.

It should be noted that even though the tampon applicator 54 has been shown as a two-piece applicator, it is possible to use a single tube applicator if desired. In a single tube applicator, only the outer tube is present and the tampon is designed to be expelled therefrom by the user's finger.

### METHOD

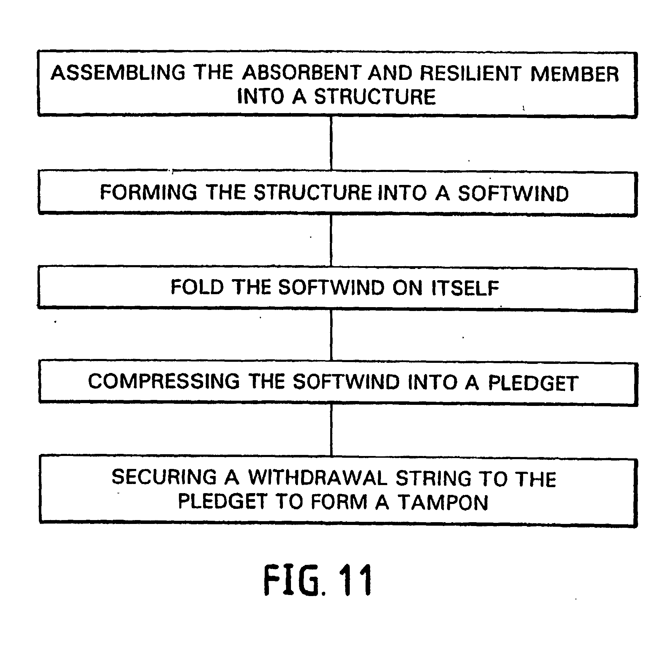

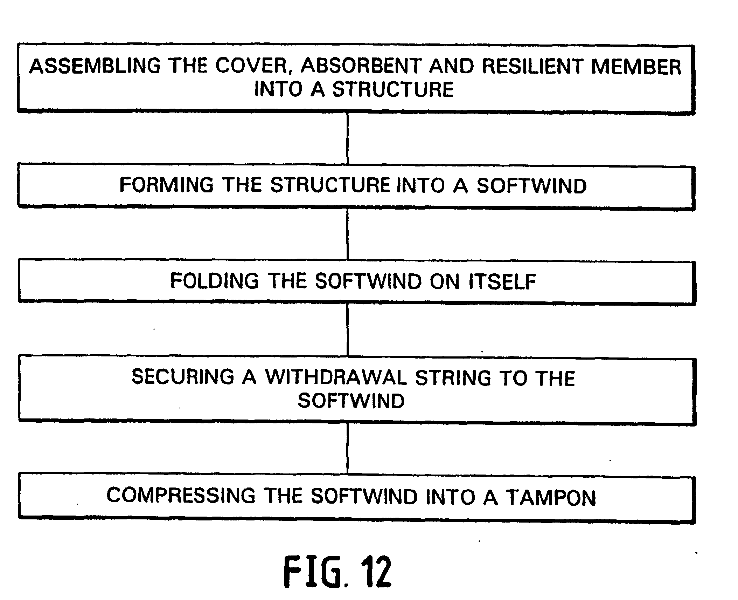

The method of forming the catamenial tampon 10 and 10' will now be explained with reference to the flow diagrams shown in Figs. 11 and 12. The method will be discussed knowing that the absorbent 12 need not be at least partially enclosed by the cover 14. The method includes the steps of assembling the resilient member 16 on the absorbent 12. By "on" is meant that the resilient member 16 is placed adjacent to or over the upper surface of the absorbent 12 such that it is vertically positioned above or situated on top of the absorbent 12. The resilient member 16 can be in physical contact with the absorbent 12 or an intermediate layer could be inserted therebetween. Preferably, the resilient member 16 will be in physical contact with the absorbent 12. The resilient member 16 and the absorbent 12 will form a structure 15 having a central longitudinal axis X--X. By "structure 15" it is meant that the resilient member 16 and the absorbent 12 can be bonded or joined together or may merely be contacting one another. Preferably, the two materials are simply arranged or vertically stacked together. However, the two materials 12 and 16 could be secured together by an adhesive, such as glue, or other known attachment means to form a laminate, if desired.

When a cover 14 is being used, the resilient member 16 is placed on the absorbent 12 such that it is spaced away from the cover 14. In other words, the resilient member 16 is positioned on the upper surface of the absorbent 12 while the cover 14 contacts the bottom surface of the absorbent 12. The resilient member 16 can be aligned along the central longitudinal axis X--X, as is shown in Fig. 1 or the resilient member 16' can be offset away from the central longitudinal axis X--X, as is shown in Fig. 3. The resilient member 16 can also be positioned closer to one end of the absorbent 12 if desired, assuming that its overall length is less than that of the absorbent 12. However, the resilient member 16 is preferably spaced an equal distance from the opposite ends of the absorbent 12.

In Fig. 1, the resilient member 16 is positioned along the central longitudinal axis X--X. The absorbent 12, the cover 14 and the resilient member 16 can be folded in half or rolled up so as to form the softwind 18. When the resilient member 16' is positioned adjacent to a longitudinal edge 17 of the absorbent 12, it makes it easier to roll up the absorbent 12, the cover 14 and the resilient member 16' to form the softwind 18'. By placing the resilient member 16' off of the centerline X--X, the resilient member 16' can be rolled up with the absorbent 12 and the cover 14 and will end up being located in the center of the softwind 18'.

Again looking at Figs. 1 and 3, one can see that the absorbent 12 and the cover 14 both have a first longitudinal edge 17 which is coterminous or contiguous with the two layers, 12 and 14 respectively. in addition, the absorbent 12 and the cover 14 are shown having contiguous edges 19 and 21, located adjacent to the opposite ends thereof. Accordingly, the absorbent 12 and the cover 14 have three coterminous edges 17, 19 and 21. Because of this, when the softwind 18 or 18' are formed, the absorbent 12 and the cover 14 will be flush with one another at the first and second ends, 22 and 24 respectively. Furthermore, the cover 14 will have a second longitudinal edge 23 which is aligned parallel with the first longitudinal edge 17 but which is outward from another longitudinal edge 25 of the absorbent 12. The reason for this is that when the absorbent 12, the cover 14 and the resilient member 16 or 16' are rolled or folded into the softwind 18 or 18', the longitudinal edge 23 of the cover 14 can overlap upon itself and be bonded thereto. This overlap 20 is shown in Figs. 2 and 4. It should be noted that it is not necessary to have edges 17, 19 and 21 be coterminous, nor to require that the longitudinal edge 23 of the cover 14 extend beyond the other longitudinal edge 25 of the absorbent 12. However, by sizing the absorbent 12 and the cover 14 as is shown in Fig. 1, it is advantageous to form the softwind 18 or 18'. When the absorbent 12, the cover 14 and the resilient member 16 or 16' are rolled up as shown in Fig. 4 to form the softwind 18', the cover 14 will circumferentially enclose the absorbent 12 and is overlapped upon itself at 20. At the overlap 20, the cover 14 can be bonded to itself by heat, pressure, heat and pressure, adhesive, etc.

As mentioned above, the absorbent 12, the cover 14 and the resilient member 16 can be formed into the softwind 18, as is shown in Fig. 2. In Fig. 2, the resilient member 16 is shorter in length than the length L₂ of the absorbent 12. Because of this length difference, the resilient member 16 will be spaced apart from the first and second ends, 22 and 24 respectively. In addition, in Fig. 2, the softwind 18 is shown being formed by folding both the absorbent 12 and the cover 14 about the resilient member 16 and then bonding the overlapped portion of the cover 20 onto itself. Alternatively, as is shown in Figs. 3 and 4, the resilient member 16' can have a length which is equal to the length L₂ of the absorbent 12. In this embodiment, the absorbent 12, the cover 14 and resilient member 16' can be rolled into the softwind 18'. It should be noted that although the method has been described with respect to folding or rolling the absorbent 12, the cover 14 and the resilient member 16 or 16' to form the softwind 18 or 18', that other means may be used for forming the softwind 18 or 18'.

The softwind 18 or 18' are then folded on themselves such that the first and second ends, 22 and 24 respectively, are aligned adjacent to one another and each of the softwinds 18 or 18' contains at least two folds 26 and 28, therebetween. The two folds 26 and 28 divide the softwind 18 or 18' into a first portion 32, a second portion 34 and a third portion 36. The first, second and third portions, 32, 34 and 36 respectively, can have an equal length or they can be of different length. For example, the second portion 34 can be of a shorter length, an equal length or a greater length than the first and third portions, 32 and 36 respectively. Furthermore, it is advantageous to form three folds 26, 28 and 30 in each of the softwinds 18 or 18' which are located between the first and second ends, 22 and 24 respectively. The three folds 26, 28 and 30 will give the softwinds 18 or 18' a generally M-shaped profile, see Fig. 5. In the generally M-shaped profile, the first and second ends, 22 and 24 are held adjacent to each other and in physical contact to one another by the withdrawal string 38. After the softwind 18 or 18' has been folded into the generally M-shaped profile, the softwind 18 or 18' can be compressed to form the pledget 44. The compressed pledget 44 has an insertion end 46 and a trailing end 48.

Referring to Fig. 11, a flow diagram is depicted showing a method of forming a tampon 10 or 10'. The method includes attaching the withdrawal string to the softwind 18 or 18' after it has been compressed into the pledget 44. An aperture 40 is then formed through the trailing end of the pledget 44 or 48. The withdrawal string 38 is threaded through the aperture 40 and is looped upon itself to hold it secure to the absorbent 12. Once the withdrawal string 38 has been added, the tampon 10 or 10' is complete. The withdrawal string 38 will facilitate removal of the tampon 10 or 10' from the woman's vagina.

Referring to Fig. 12, another flow diagram is depicted, which shows an alternative way to attach the withdrawal string 38. In this method, the aperture 40 is formed through the first and second ends, 22 and 24 respectively, of the softwind 18 or 18'. The withdrawal string 38 is then threaded through the aperture 40 and looped upon itself so as to secure the withdrawal string 38 to the softwind 18 or 18'. After the withdrawal string 38 has been added, the softwind 18 or 18' is compressed into the pledget 44.

It should be noted that in the above two methods, when the softwind 18 or 18' is compressed into the pledget 44, the insertion end 46 could contain more absorbent material than the trailing end 48. This additional absorbent material at the insertion end 46 is advantageous in intercepting fluid flow and preventing premature leakage of body fluid past the tampon 10 or 10' immediately after the tampon 10 or 10' has been inserted into the woman's vagina.

While the invention has been described in conjunction with several specific embodiments, it is to be understood that many alternatives, modifications and variations will be apparent to those skilled in the art in light of the aforegoing description. Accordingly, this invention is intended to embrace all such alternatives, modifications and variations which fall within the scope of the appended claims.

## Claims

1. A tampon (10) comprising:
a) an absorbent (12) and withdrawal means (38) for removing said tampon (10) from a woman's vagina, and a softwind having a first end and a second end (22, 24), said softwind (18) having been folded upon itself such that said first and second ends (22, 24) are aligned adjacent to one another and said softwind (18) contains at least two folds (26, 28) therebetween, said softwind (18) having been compressed into an elongated pledget (44) having an insertion end (46) and a trailing end (48) **characterized in that** it further comprises
b) a resilient member (16) positioned on said absorbent (12), said absorbent (12) and said resilient member (16) having been formed into the elongated softwind (18), said resilient member (16) capable of expanding at least a portion of said pledget (44) to intercept fluid flow when inserted into a woman's vagina and the withdrawal means (38) being attached to first and second ends (22, 24) of a softwind (18).

2. The tampon (10) of claim 1 wherein said softwind (18) is folded at two locations to divide said softwind (18) in a first portion (32), a second portion (34) and a third portion (36).

3. The tampon (10) of claim 2 wherein said first portion (32) and said third portions (36) are approximately the same length.

4. The tampon (10) of claim 2 wherein said insertion end (46) of said pledget (44) contains more absorbent than said trailing end (48) of said pledget (44).

5. The tampon (10) of claim 1 wherein said resilient member (16) extends between said two folds (26, 28).

6. The tampon (10) of claim 1 wherein said resilient member (16) extends beyond said two folds (26, 28).

7. The tampon (10) of claim 1 wherein said softwind (18) contains three folds (26, 28, 30) and has a generally M-shaped profile before being compressed.

8. The tampon (10) of claim 2 wherein said withdrawal means (38) is a withdrawal string which passes through said first and third portions (32, 36) adjacent to said first and second ends (22, 24) of said softwind (18).

9. The tampon (10) of claim 1 wherein said resilient member (16) is foam.

10. The tampon (10) of claim 1 wherein said resilient member (16) is rolled into the elongated softwind (18), wherein said softwind (18) contains at least three folds (26, 28, 30) therebetween to form a generally M-shaped profile, and wherein said insertion end (46) contains more absorbent than said trailing end (48).

11. The tampon (10) of claim 10 wherein said resilient member (16) is spaced inward from said first and second ends (22, 24) of said softwind (18).

12. The tampon (10) of claim 10 wherein said resilient member (16) is a closed cell foam.

13. The tampon (10) of claim 10 wherein said resilient member (16) is an open cell foam.

14. The tampon (10) of claim 10 wherein said resilient member (16) is a wettable foam.

15. The tampon (10) of claim 10 wherein said resilient member (16) extends beyond said three folds (26, 28, 30).

16. The tampon (10) of claim 1 which is designed to be inserted into a woman's vagina, said tampon (10) further comprising:
a liquid-permeable cover (14) circumferentially surrounding at least a portion of said absorbent (12), said cover (14) having been bonded to said absorbent (12), wherein said absorbent (12) and said resilient member (16) have been formed together with said cover (14) into the elongated softwind (18) and wherein said resilient member (16) is spaced inward from said first and second ends, wherein said softwind (18) contains three folds (26, 28, 30) therebetween to form a generally M-shaped profile, wherein said softwind (18) has been compressed into a cylindrically shaped pledget (44) and wherein said resilient member (16) extends beyond said three folds (26, 28, 30).

17. The tampon (10) of claim 16 wherein said tampon (10) is retained in an applicator tube (54) prior to use and upon insertion of said tampon (10) into a woman's vagina said resilient member (16) causes at least a portion of said pledget (44) to expand outwardly against the walls of the woman's vagina.

18. The tampon (10) of claim 16 wherein said tampon (10) has a triangular shape when expanded by said resilient member (16).

19. The tampon (10) of claim 16 wherein said absorbent (12), cover (14) and resilient member (16) have been radially compressed into a cylindrically shaped pledget (44) having first and second ends (22, 24) which are held in direct contact with one another by said withdrawal means (38).

20. The tampon (10) of claim 16 wherein said insertion end (46) of said pledget (44) contains more absorbent than said trailing end (48) of said pledget (44).

21. A method of forming the tampon (10) of claim 1, said method comprising the steps of:
a) placing a resilient member (16) on an absorbent (12) to form a structure having a central longitudinal axis, said resilient member (16) being aligned along said central longitudinal axis;
b) forming said absorbent (12) and resilient member (16) into an elongated softwind (18) having a first end and a second end (22, 24).
c) folding said softwind (18) on itself such that said first and second ends (22, 24) are aligned adjacent to one another and said softwind (18) contains at least two folds (26, 28) therebetween;
d) compressing said softwind (18) into a pledget (44) having an insertion end (46) and a trailing end (48), and
e) securing a withdrawal string (38) to first and second ends of said pledget (44) to form a tampon (10), said withdrawal string (38) facilitating removal of said tampon (10) from a woman's vagina.

22. The method of claim 21 wherein said softwind contains two folds (26, 28), wherein said two folds (26, 28) divide said softwind (18) into a first portion (32), a second portion (34) and a third portion (36) and said second portion (34) is shorter in length than said first portion (32).

23. The method of claim 22 wherein said two folds (26, 28) divide said softwind (18) into a first portion (32), a second portion (34) and a third portion (36) and said first and third portions (32, 36) have equal lengths.

24. The method of claim 21 wherein said softwind (18) contains three folds (26, 28, 30) located between said first and second ends (22, 24).

25. The method of claim 24 wherein said softwind (18) contains three folds (26, 28, 30) giving it an M-shaped profile.

26. The method of claim 25 wherein said first and second ends (22, 24) of said softwind (18) are held in contact with one another by said withdrawal string (38).

27. The method of claim 21 wherein said absorbent (12) and said resilient member (16) are rolled to form said softwind (18).

28. The method of claim 21, said method in advance of step a) of claim 21 further comprising the step of:
placing the absorbent (12) on a cover (14) to form the structure having a central longitudinal axis in advance of step a) of claim 21, said cover (14) and said absorbent (12) having at least one contiguous edge;
wherein said resilient member (16) is placed on said structure, and is spaced apart from said cover (14), and wherein said absorbent (12) and resilient member (16) are rolled together with said cover (14).

29. The method of claim 28 wherein said insertion end (46) contains more absorbent than said trailing end (48).

30. The method of claim 28 wherein said softwind (18) contains three folds (26, 28, 30) giving it an M-shaped profile.

31. The method of claim 28 wherein said absorbent (12) and said cover (14) have three coterminous edges.

32. A method of forming the tampon (10) of claim 1, said method comprising the steps of:
a) placing an absorbent (12) on a cover (14) to form a structure having a central longitudinal axis;
b) placing a resilient member (16) on said structure, said resilient member (16) being aligned parallel to and offset from said central longitudinal axis, and said resilient member (16) being spaced apart from said cover (14);
c) forming said cover (14), absorbent (12) and resilient member (16) into an elongated softwind (18) having a first end and a second end (22, 24);
d) folding said softwind (18) on itself such that said first and second ends (22, 24) are aligned adjacent to one another and said softwind (18) contains three folds (26, 28, 30) therebetween;
e) compressing said softwind (18) into a pledget (44) having an insertion end (46) and a trailing end (48), said insertion end (46) containing more absorbent than said trailing end (48); and
f) securing a withdrawal string (38) to first and second ends of said pledget (44) to form a tampon (10), said withdrawal string (38) facilitating removal of said tampon (10) from a woman's vagina.

33. The method of claim 32 wherein an aperture (40) is formed in said first and second ends (22, 24) of said softwind (18) and said withdrawal string (38) is passed through said aperture (40) and looped upon itself to hold said withdrawal string (38) secure thereto.

34. The method of claim 32 wherein said cover (14), absorbent (12) and resilient member (16) are folded into an elongated softwind (18).

35. The method of claim 32 wherein said resilient member (16) has a shorter length than said absorbent (12).

36. The method of claim 32 wherein said resilient member (16) has a length equal to that of said absorbent (12).

37. A method of forming the tampon (10) of claim 1, said method comprising the steps of:
a) placing an absorbent (12) on a liquid-permeable cover (14) to form a structure having a central longitudinal axis, said cover (14) and said absorbent (12) having at least one contiguous edge;
b) placing a resilient member (16) on said structure, said resilient member (16) being offset from said central longitudinal axis and being spaced apart from said cover (14);
c) forming said cover (14), absorbent (12) and resilient member (16) into an elongated softwind (18) having a first end and a second end (22, 24);
d) folding said softwind (18) on itself such that said first and second ends (22, 24) are aligned adjacent to one another and said softwind (18) contains three folds (26, 28, 30) therebetween which give it an M-shaped profile;
e) securing a withdrawal string (38) to said first and second ends of softwind (18), said withdrawal string (38) facilitating removal of said tampon (10) from a woman's vagina; and
f) compressing said softwind (18) into a tampon (10).

38. The method of claim 37 wherein an aperture (40) is formed in said first and second ends (22, 24) of said softwind (18) and said withdrawal string (38) is passed through said aperture (40) and looped upon itself to hold said withdrawal string (38) secure thereto.

39. The method of claim 37 wherein said absorbent (12) has a pair of spaced apart longitudinal edges and said resilient member (16) is placed adjacent to one of said longitudinal edges.

40. The method of claim 37 wherein said resilient member (16) has a length equal to that of said absorbent (12).

## Patentansprüche

1. Tampon (10) umfassend:
a) ein Absorbens (12) und Rückziehmittel (38), um den Tampon (10) aus der Vagina einer Frau zu entfernen, und einen Weichwickel mit einem ersten Ende und einem zweiten Ende (22, 24), wobei der Weichwickel (18) auf sich selbst gefaltet wurde, so dass die ersten und zweiten Enden (22, 24) einander benachbart sind, und wobei der Weichwickel (18) wenigstens zwei Falten (26, 28) dazwischen enthält, wobei der Weichwickel (18) zu einem verlängerten Bausch (44) mit einem Einführende (46) und einem Nachlaufende (48) gepresst wurde,
**gekennzeichnet dadurch, dass** es ferner umfasst:
b) ein elastisches Element (16), welches auf dem Absorbens (12) angeordnet ist, wobei das Absorbens (12) und das elastische Element (16) zu dem verlängerten Weichwickel (18) gebildet wurden, wobei das elastische Element (16) zum Ausdehnen von wenigstens einem Bereich des Bausches (44) geeignet ist, um Fluidfluss zu unterbrechen, wenn er in die Vagina einer Frau eingeführt ist, und wobei die Rückziehmittel (38) an ersten und zweiten Enden (22, 24) des Weichwickels (18) angebracht sind.

2. Tampon (10) gemäß Anspruch 1, wobei der Weichwickel (18) an zwei Stellen gefaltet ist, um den Weichwickel (18) in einen ersten Bereich (32), einen zweiten Bereich (34) und einen dritten Bereich (36) zu unterteilen.

3. Tampon (10) gemäß Anspruch 2, wobei der erste Bereich (32) und der dritte Bereich (36) ungefähr dieselbe Länge haben.

4. Tampon (10) gemäß Anspruch 2, wobei das Einführende (46) des Bausches (44) mehr Absorbens enthält als das Nachlaufende (48) des Bausches (44).

5. Tampon (10) gemäß Anspruch 1, wobei das elastische Element (16) sich zwischen den zwei Falten (26, 28) erstreckt.

6. Tampon (10) gemäß Anspruch 1, wobei das elastische Element (16) sich über die zwei Falten (26, 28) hinaus erstreckt.

7. Tampon (10) gemäß Anspruch 1, wobei der Weichwickel (18) drei Falten (26, 28, 30) enthält und im Allgemeinen ein M-förmiges Profil hat bevor sie gepresst wird.

8. Tampon (10) gemäß Anspruch 2, wobei das Rückholmittel (38) eine Rückholschnur ist, welches durch die ersten und dritten Bereiche (32, 36) benachbart den ersten und zweiten Enden (22, 24) des Weichwickels (18) läuft.

9. Tampon (10) gemäß Anspruch 1, wobei das elastische Element (16) Schaum ist.

10. Tampon (10) gemäß Anspruch 1, wobei das elastische Element (16) zu dem verlängerten Weichwickel (18) gerollt ist, wobei der Weichwickel (18) wenigstens drei Falten (26, 28, 30) dazwischen enthält, um ein im Allgemeinen M-förmiges Profil zu bilden, und wobei das Einführende (46) mehr Absorbens enthält als das Nachlaufende (48).

11. Tampon (10) gemäß Anspruch 10, wobei das elastische Element (16) von dem ersten und zweiten Enden (22, 24) des Weichwickels (18) nach innen beabstandet ist.

12. Tampon (10) gemäß Anspruch 10, wobei das elastische Element (16) ein Geschlossen-Zell-Schaum ist.

13. Tampon (10) gemäß Anspruch 10, wobei das elastische Element (16) ein Offen-Zell-Schaum ist.

14. Tampon (10) gemäß Anspruch 10, wobei das elastische Element (16) ein benetzbarer Schaum ist.

15. Tampon (10) gemäß Anspruch 10, wobei sich das elastische Element (16) über die drei Falten (26, 28, 30) hinaus erstreckt.

16. Tampon (10) gemäß Anspruch 1, welches zum Einführen in die Vagina einer Frau ausgebildet ist, wobei der Tampon (10) ferner umfasst:
eine flüssigkeitsdurchlässige Abdeckung (14), welche wenigstens einen Bereich des Absorbens (12) am Umfang umgibt, wobei die Abdeckung (14) an das Absorbens (12) bondiert wurde, wobei das Absorbens (12) und das elastische Element (16) zusammen mit der Abdeckung (14) zu einem verlängerten Weichwickel (18) gebildet wurden, und wobei das elastische Element (16) von den ersten und zweiten Enden nach innen beabstandet ist,
wobei der Weichwickel (18) drei Falten (26, 28, 30) dazwischen umfasst, um ein im Wesentlichen M-förmiges Profil zu haben, wobei der Weichwickel (18) zu einem zylindrisch geformten Bausch (44) zusammengedrückt wurde, und wobei sich das elastische Element (16) über die drei Falten (26, 28, 30) hinaus erstreckt.

17. Tampon (10) gemäß Anspruch 16, wobei der Tampon (10) vor der Verwendung und bis zur Einführung des Tampons (10) in die Vagina einer Frau in einer Applikatorröhre (54) zurückgehalten wird, wobei das elastische Element (16) verursacht, dass wenigstens ein Bereich des Bausches (44) sich nach außen gegen die Wände der Vagina einer Frau ausdehnt.

18. Tampon (10) gemäß Anspruch 16, wobei der Tampon (16) eine dreieckige Gestalt hat, wenn es durch das elastische Element (16) ausgedehnt ist.

19. Tampon (10) gemäß Anspruch 16, wobei das Absorbens (12), die Abdeckung (14) und das elastische Element (16) radial zu einem zylindrisch geformten Bausch (44) mit ersten und zweiten Enden (22, 24) zusammengedrückt wurde, welche in direktem Kontakt zueinander durch die Rückziehmittel (38) gehalten sind.

20. Tampon (10) gemäß Anspruch 16, wobei das Einführende (46) des Bausches (44) mehr Absorbens enthält als das Nachlaufende (48) des Bausches (44).

21. Verfahren zum Bilden des Tampons (10) gemäß Anspruch 1, wobei das Verfahren die folgenden Schritte umfasst:
a) Anordnen eines elastischen Elements (16) auf einem Absorbens (12), um eine Struktur mit einer längsverlaufenden Mittelachse zu bilden, wobei das elastische Element (16) entlang der längsverlaufenden Mittelachse angeordnet ist;
b) Formen des Absorbens (12) und des elastischen Elements (16) zu einem verlängerten Weichwickel (18) mit einem ersten Ende und einem zweiten Ende (22, 24);
c) Falten des Weichwickels (18) auf sich selbst, so dass die ersten und zweiten Enden (22, 24) einander benachbart angeordnet sind und der Weichwickel (18) wenigstens zwei Falten (26, 28) dazwischen enthält;
d) Zusammendrücken des Weichwickels (18) zu einem Bausch (44) mit einem Einführende (46) und einem Nachlaufende (48), und
e) Haltern der Rückziehschnur (38) an den ersten und zweiten Enden des Bausches (44), um einen Tampon (10) zu bilden, wobei die Rückziehschnur (38) die Entfernung des Tampons (10) aus der Vagina einer Frau erleichtert.

22. Verfahren gemäß Anspruch 21, wobei der Weichwickel zwei Falten (26, 28) enthält, wobei die zwei Falten (26, 28) den Weichwickel (18) in einen ersten Bereich (32), einen zweiten Bereich (34) und einen dritten Bereich (36) unterteilen, und wobei der zweite Bereich (34) in der Länge kürzer ist als der erste Bereich (32).

23. Verfahren gemäß Anspruch 22, wobei die zwei Falten (26, 28) den Weichwickel (18) in einen ersten Bereich (32), einen zweiten Bereich (34) und einen dritten Bereich (36) unterteilen, und wobei die ersten und dritten Bereiche (32, 36) gleiche Längen haben.

24. Verfahren gemäß Anspruch 21, wobei der Weichwickel drei Falten (26, 28, 30) enthält, welche zwischen den ersten und zweiten Enden (22, 24) angeordnet sind.

25. Verfahren gemäß Anspruch 24, wobei der Weichwickel drei Falten (26, 28, 30) enthält, welche ihm ein M-förmiges Profil geben.

26. Verfahren gemäß Anspruch 25, wobei die ersten und zweiten Enden (22, 24) des Weichwickels (18) durch die Rückziehschnur (38) in Berührung miteinander gehalten werden.

27. Verfahren gemäß Anspruch 21, wobei das Absorbens (12) und das elastische Element (16) gerollt werden, um den Weichwickel (18) zu bilden.

28. Verfahren gemäß Anspruch 21, wobei das Verfahren vor Schritt a) gemäß Anspruch 21 den folgenden Schritt umfasst:
Anordnen des Absorbens (12) auf einer Abdeckung (14), um die Struktur mit einer längsverlaufenden Mittelachse vor dem Schritt a) gemäß Anspruch 21 zu bilden, wobei die Abdeckung (14) und das Absorbens (12) wenigstens einen fortlaufenden Rand haben;
wobei das elastische Element (16) auf der Struktur angeordnet ist und von der Abdeckung (14) beabstandet ist, und wobei das Absorbens (12) und das elastische Element (16) zusammen mit der Abdeckung (14) aufgerollt werden.

29. Verfahren gemäß Anspruch 28, wobei das Einführende (46) mehr Absorbens enthält als das Nachlaufende (48).

30. Verfahren gemäß Anspruch 28, wobei der Weichwickel (18) drei Falten (26, 28, 30) enthält, welche ihm ein M-förmiges Profil geben.

31. Verfahren gemäß Anspruch 28, wobei das Absorbens (12) und die Abdeckung (14) drei miteinander abschliessende Ränder haben.

32. Verfahren zum Bilden des Tampons (10) gemäß Anspruch 1, wobei das Verfahren die folgenden Schritte umfasst:
a) Anordnen eines Absorbens (12) auf einer Abdeckung (14), um eine Struktur mit einer längsverlaufenden Mittelachse zu bilden;
b) Anordnen eines elastischen Elements (16) auf der Struktur, wobei das elastische Element (16) parallel zu der längsverlaufenden Mittelachse versetzt angeordnet ist, und wobei das elastische Element (16) von der Abdeckung (14) beabstandet ist;
c) Formen der Abdeckung (14), des Absorbens (12) und des elastischen Elements (16) zu einem verlängerten Weichwickel (18) mit einem ersten Ende und einem zweiten Ende (22, 24);
d) Falten des Weichwickels (18) auf sich selbst, so dass die ersten und zweiten Enden (22, 24) einander benachbart angeordnet sind und der Weichwickel (18) drei Falten (26, 28, 30) dazwischen enthält;
e) Zusammendrücken des Weichwickels (18) zu einem Bausch (44) mit einem Einführende (46) und einem Nachlaufende (48), wobei das Einführende (46) mehr Absorbens enthält als das Nachlaufende (48); und
f) Haltern einer Rückziehschnur (38) an den ersten und zweiten Enden des Bausches (44), um einen Tampon (10) zu bilden, wobei die Rückziehschnur (38) die Entfernung des Tampons (10) aus der Vagina einer Frau erleichtert.

33. Verfahren gemäß Anspruch 32, wobei eine Öffnung (40) in den ersten und zweiten Enden (22, 24) des Weichwickels (18) gebildet ist und die Rückziehschnur (38) durch die Öffnung (40) läuft und um sich selbst geschlungen ist, um die Rückziehschnur (38) daran zu befestigen.

34. Verfahren gemäß Anspruch 32, wobei die Abdeckung (14), das Absorbens (12) und das elastische Element (16) zu einem verlängerten Weichwickel (18) gefaltet werden.

35. Verfahren gemäß Anspruch 32, wobei das elastische Element (16) eine kürzere Länge hat als das Absorbens (12).

36. Verfahren gemäß Anspruch 32, wobei das elastische Element (16) dieselbe Länge hat wie die des Absorbens (12).

37. Verfahren zum Bilden des Tampons (10) gemäß Anspruch 1, wobei das Verfahren die folgenden Schritte umfasst:
a) Anordnen eines Absorbens (12) auf einer flüssigkeitsdurchlässigen Abdeckung (14), um eine Struktur mit einer längsverlaufenden Mittelachse zu bilden, wobei die Abdeckung (14) und das Absorbens (12) wenigstens einen fortlaufenden Rand haben;
b) Anordnen eines elastischen Elements (16) auf der Struktur, wobei das elastische Element (16) von der längsverlaufenden Mittelachse versetzt ist und von der Abdeckung (14) beabstandet ist;
c) Formen der Abdeckung (14), des Absorbens (12) und des elastischen Elements (16) zu einem verlängerten Weichwickel (18) mit einem ersten Ende und einem zweiten Ende (22, 24);
d) Falten des Weichwickels (18) auf sich selbst, so dass die ersten und zweiten Enden (22, 24) einander benachbart angeordnet sind und der Weichwickel (18) drei Falten (26, 28, 30) dazwischen enthält, welche ihm ein M-förmiges Profil geben;
e) Haltern einer Rückziehschnur (38) an den ersten und zweiten Enden des Weichwickels (18), wobei die Rückziehschnur (38) die Entfernung des Tampons (10) aus der Vagina einer Frau erleichtert; und
f) Zusammendrücken des Weichwickels (18) zu einem Tampon (10).

38. Verfahren gemäß Anspruch 37, wobei eine Öffnung (40) in den ersten und zweiten Enden (22, 24) des Weichwickels (18) gebildet ist und die Rückziehschnur (38) durch die Öffnung (40) geführt ist und um sich selbst geschlungen ist, um die Rückziehschnur (38) daran zu befestigen.

39. Verfahren gemäß Anspruch 37, wobei das Absorbens (12) ein Paar beabstandeter längsverlaufender Ränder hat und das elastische Element (16) benachbart eines der längsverlaufenden Ränder angeordnet ist.

40. Verfahren gemäß Anspruch 37, wobei das elastische Element (16) eine gleiche Länge hat wie das Absorbens (12).

## Revendications

1. Tampon (10) comprenant :
a) un absorbant (12) et un moyen d'extraction (38) pour extraire ledit tampon (10) du vagin d'une femme et un enroulement souple (18) ayant une première extrémité et une seconde extrémité (22,24), ledit enroulement souple (18) ayant été replié sur lui-même de telle sorte que lesdites première et seconde extrémités (22,24) sont alignées adjacentes l'une à l'autre, et ledit enroulement souple (18) comporte, entre lesdites extrémités, au moins deux plis (26,28), ledit enroulement souple (18) ayant été comprimé en une compresse (44) allongée ayant une extrémité d'insertion (46) et une extrémité de fuite (48),
caractérisé en qu'il comprend en outre
b) un élément résilient (16) positionné sur ledit absorbant (12), ledit absorbant (12) et ledit élément résilient (16) ayant été conformés en ledit enroulement souple (18) allongé, ledit élément résilient (16) étant capable de dilater au moins une portion de ladite compresse (44) pour intercepter le flux de fluides lorsqu'il est inséré dans le vagin d'une femme et le moyen d'extraction (38) étant fixé aux première et seconde extrémités (22,24) dudit enroulement souple (18).

2. Tampon (10) selon la revendication 1, dans lequel ledit enroulement souple (18) est replié en deux emplacements pour diviser ledit enroulement souple (18) en une première portion (32), une seconde portion (34) et une troisième portion (36).

3. Tampon (10) selon la revendication 2, dans lequel ladite première portion (32) et ladite troisième portion (36) sont approximativement de même longueur.

4. Tampon (10) selon la revendication 2, dans lequel ladite extrémité d'insertion (46) de ladite compresse (44) contient davantage d'absorbant que ladite extrémité de fuite (48) de ladite compresse (44).

5. Tampon (10) selon la revendication 1, dans lequel ledit élément résilient (16) s'étend entre lesdits deux plis (26,28).

6. Tampon (10) selon la revendication 1, dans lequel ledit élément résilient (16) s'étend au-delà desdits deux plis (26,28).

7. Tampon (10) selon la revendication 1, dans lequel ledit enroulement souple (18) comporte trois plis (26,28,30) et a un profil généralement en forme de M avant d'être comprimé.

8. Tampon (10) selon la revendication 2, dans lequel ledit moyen d'extraction (38) est un cordon d'extraction qui passe au travers desdites première et troisième portions (32,36) en une zone adjacente auxdites première et seconde extrémités (22,24) dudit enroulement souple (18).

9. Tampon (10) selon la revendication 1, dans lequel ledit élément résilient (16) est une mousse.

10. Tampon (10) selon la revendication 1, dans lequel ledit élément résilient (16) est roulé en ledit enroulement souple (18) allongé, ledit enroulement souple (18) comportant au moins trois plis (26,28,30) intermédiaire pour former un profil généralement en M, et dans lequel ladite extrémité d'insertion (46) contient davantage d'absorbant que ladite extrémité de fuite (48).

11. Tampon (10) selon la revendication 10, dans lequel ledit élément résilient (16) est espacé, vers l'intérieur, desdites première et seconde extrémités (22,24) dudit enroulement souple (18).

12. Tampon (10) selon la revendication 10, dans lequel ledit élément résilient (16) est une mousse à cellule fermée.

13. Tampon (10) selon la revendication 10, dans lequel ledit élément résilient (16) est une mousse à cellule ouverte.

14. Tampon (10) selon la revendication 10, dans lequel ledit élément résilient (16) est une mousse mouillable.

15. Tampon (10) selon la revendication 10, dans lequel ledit élément résilient (16) s'étend au-delà desdits trois plis (26,28,30).

16. Tampon (10) selon la revendication 1, qui est conçu pour être inséré dans le vagin d'une femme, ledit tampon (10) comprenant en outre :
une enveloppe (14) perméable aux liquides entourant circonférentiellement au moins une portion dudit absorbant (12), ladite enveloppe (14) ayant été liée audit absorbant (12), ledit absorbant (12) et ledit élément résilient (16) ayant été conformés ensemble, avec ladite enveloppe (14), en un enroulement souple (18) allongé, ledit élément résilient (16) étant espacé vers l'intérieur desdites première et seconde extrémités, ledit enroulement souple (18) comportant, entre elles, trois plis (26,28,30) pour former un profil généralement en forme de M, ledit enroulement souple (18) ayant été comprimé en une compresse (44) de forme cylindrique et ledit élément résilient (16) s'étendant au-delà desdits trois plis (26,28,30).

17. Tampon (10) selon la revendication 16, qui est retenu dans un tube applicateur (54) avant d'être d'utilisé et, lors de l'insertion dudit tampon (10) dans le vagin d'une femme, ledit élément résilient (16) fait qu'au moins une portion de ladite compresse (44) se dilate vers l'extérieur contre les parois du vagin de la femme.

18. Tampon (10) selon la revendication 16, qui a une forme triangulaire lorsqu'il est dilaté par ledit élément résilient (16).

19. Tampon (10) selon la revendication 16, dans lequel ledit absorbant (12), ladite enveloppe (14) et ledit élément résilient (16) ont été comprimés radialement en une compresse (44) de forme cylindrique ayant des première et seconde extrémités (22,24) qui sont maintenues en contact direct l'une avec l'autre par ledit moyen d'extraction (38).

20. Tampon (10) selon la revendication 16, dans lequel ladite extrémité d'insertion (46) de ladite compresse (44) contient davantage d'absorbant que ladite extrémité de fuite (48) de ladite compresse (44).

21. Procédé de formation du tampon (10) selon la revendication 1, ledit procédé comprenant les étapes suivantes :
a) le positionnement d'un élément résilient (16) sur un absorbant (12) pour former une structure ayant un axe longitudinal central, ledit élément résilient (16) étant aligné le long dudit axe longitudinal central ;
b) la conformation dudit absorbant (12) et dudit élément résilient (16) en un enroulement souple (18) allongé ayant une première extrémité et une seconde extrémité (22,24) ;
c) le pliage dudit enroulement souple (18) sur lui-même de telle sorte que lesdites première et seconde extrémités (22,24) sont alignées adjacentes l'une à l'autre et ledit enroulement souple (18) comporte, en elles, au moins deux plis (26,28) ;
d) la compression dudit enroulement souple (18) en une compresse (44) ayant une extrémité d'insertion (46) et une extrémité de fuite (48) ; et
e) la fixation d'un cordon d'extraction (38) aux première et seconde extrémités de ladite compresse (44) pour former un tampon (10), ledit cordon d'extraction (38) facilitant l'extraction dudit tampon (10) depuis le vagin de la femme.

22. Procédé selon la revendication 21, dans lequel ledit enroulement souple comporte deux plis (26,28), lesdits deux plis (26,28) divisant ledit enroulement souple (18) en une première portion (32), une seconde portion (34) et une troisième portion (36), ladite seconde portion (34) étant plus courte que ladite première portion (32).

23. Procédé selon la revendication 22, dans lequel lesdits deux plis (26,28) divisent ledit enroulement souple (18) en une première portion (32), une seconde portion (34) et une troisième portion (36), lesdites première et troisième portions (32,36) étant d'égales longueurs.

24. Procédé selon la revendication 21, dans lequel ledit enroulement souple (18) comporte trois plis (26,28,30) situés entre lesdites première et seconde extrémités (22,24).

25. Procédé selon la revendication 24, dans lequel ledit enroulement souple (18) comporte trois plis (26,28,30), lui donnant un profil en M.

26. Procédé selon la revendication 25, dans lequel lesdites première et seconde extrémités (22,24) dudit enroulement souple (18) sont maintenues en contact l'une avec l'autre par ledit cordon d'extraction (38).

27. Procédé selon la revendication 21, dans lequel ledit absorbant (12) et ledit élément résilient (16) sont enroulés pour former ledit enroulement souple (18).

28. Procédé selon la revendication 21, ledit procédé comportant comme étape antérieure à l'étape a) de la revendication 21 :
le positionnement de l'absorbant (12) sur une enveloppe (14) pour former la structure ayant un axe central longitudinal préalablement à l'étape a) de la revendication 21, ladite enveloppe (14) et ledit absorbant (12) ayant au moins un bord contigu ;
ledit élément résilient (16) étant placé sur ladite structure et espacé de ladite enveloppe (14), ledit absorbant (12) et ledit élément résilient (16) étant roulés ensemble avec ladite enveloppe (14).

29. Procédé selon la revendication 28, dans lequel ladite extrémité d'insertion (46) contient davantage d'absorbant que ladite extrémité de fuite (48).

30. Procédé selon la revendication 28, dans lequel ledit enroulement souple (18) comporte trois plis (26,28,30) lui donnant un profil en forme de M.

31. Procédé selon la revendication 28, dans lequel ledit absorbant (12) et ladite enveloppe (14) ont trois bords coterminaux.

32. Procédé de formation du tampon (10) selon la revendication 1, ledit procédé comprenant les étapes suivantes :
a) le positionnement d'un absorbant (12) sur une enveloppe (14) pour former une structure ayant un axe central longitudinal ;
b) le positionnement d'un élément résilient (16) sur ladite structure, ledit élément résilient (16) étant aligné parallèlement audit axe central longitudinal et en en étant décalé, ledit élément résilient (16) étant espacé de ladite enveloppe (14) ;
c) la conformation de ladite enveloppe (14) dudit absorbant (12) et dudit élément résilient (16) en un enroulement souple (18) allongé ayant une première extrémité et une seconde extrémité (22,24) ;
d) le pliage dudit enroulement souple (18) sur lui-même de telle sorte que lesdites première et seconde extrémités (22,24) sont alignées adjacentes l'une à l'autre et ledit enroulement souple (18) comporte, entre elles, trois plis (26,28,30) ;
e) la compression dudit enroulement souple (18) en une compresse (44) ayant une extrémité d'insertion (46) et une extrémité de fuite (48), ladite extrémité d'insertion (46) contenant davantage d'absorbant que ladite extrémité de fuite (48) ; et
f) la fixation d'un cordon d'extraction (38) aux première et seconde extrémités de ladite compresse (44) pour former un tampon (10), ledit cordon d'extraction (38) facilitant l'extraction dudit tampon (10) depuis un vagin féminin.

33. Procédé selon la revendication 32, dans lequel une ouverture (40) est formée dans lesdites première et seconde extrémités (22,24) dudit enroulement souple (18) et ledit cordon d'extraction (38) est passé au travers de ladite ouverture (40) et refermé en boucle sur lui-même pour maintenir ledit cordon d'extraction (38) fixé audit enroulement.

34. Procédé selon la revendication 32, dans lequel ladite enveloppe (14), ledit absorbant (12) et ledit élément résilient (16) sont pliés en un enroulement souple (18) allongé.

35. Procédé selon la revendication 32, dans lequel ledit élément résilient (16) est plus court que ledit absorbant (12).

36. Procédé selon la revendication 32, dans lequel ledit élément résilient (16) a une longueur égale à celle dudit absorbant (12).

37. Procédé de formation du tampon (10) selon la revendication 1, ledit procédé comprenant les étapes suivantes :
a) le positionnement d'un absorbant (12) sur une enveloppe (14) perméable aux liquides pour former une structure ayant un axe central longitudinal, ladite enveloppe (14) et ledit absorbant (12) ayant au moins un bord contigu ;
b) le positionnement d'un élément résilient (16) sur ladite structure, ledit élément résilient (16) étant décalé dudit axe central longitudinal et étant espacé de ladite enveloppe (14) ;
c) la conformation de ladite enveloppe (14), dudit absorbant (12) et dudit élément résilient (16) en un enroulement souple (18) allongé ayant une première extrémité et une seconde extrémité (22,24) ;
d) le pliage dudit enroulement souple (18) sur lui-même de telle sorte que lesdites première et seconde extrémités (22,24) soient alignées adjacentes l'une à l'autre et ledit enroulement souple (18) comporte, entre elles, trois plis (26,28,30) pour donner un profil en forme de M ;
e) la fixation d'un cordon d'extraction (38) auxdites première et seconde extrémités de l'enroulement souple (18), ledit cordon d'extraction (38) facilitant l'extraction dudit tampon (10) du vagin d'une femme ; et
f) la compression dudit enroulement souple (18) en un tampon (10).

38. Procédé selon la revendication 37, dans lequel une ouverture (40) est formée dans lesdites première et seconde extrémités (22,24) dudit enroulement souple (18) et ledit cordon d'extraction (38) est passé au travers de ladite ouverture (40) et refermé en boucle sur lui-même pour retenir ledit cordon d'extraction (38) fixé audit enroulement.

39. Procédé selon la revendication 37, dans lequel ledit absorbant (12) comporte une paire de bords longitudinaux espacés et ledit élément résilient (16) est placé adjacent à l'un desdits bords longitudinaux.

40. Procédé selon la revendication 37, dans lequel ledit élément résilient (16) a une longueur égale à celle dudit absorbant (12).
